# EUROPEAN PATENT APPLICATION

(11) **EP 4 678 178 A1**
(43) Date of publication of application: **14.01.2026**
(21) Application number: 24188082.2
(22) Date of filing: 11.07.2024
(51) Int. Cl.: A61K 33/243, A61K 39/395, A61P 35/00, C07K 16/22

(54) **METHOD OF INHIBITION, PREVENTION AND TREATMENT OF CANCER METASTASIS**

(71) Applicant: Smadmin Therapeutics B.V., 2012 JA Haarlem (NL)
(72) Inventor: Krishnadath, Kausilia Krishnawatie, 2012 JA Haarlem (NL)
(74) Representative: De Vries & Metman

(57) **Abstract**

The present invention relates to methods for inhibiting, preventing, and treating cancer metastasis in patients using anti-BMP4 antibodies. Anti-BMP4 antibodies have been found to inhibit and even prevent cancer metastasis in patient. These antibodies have also been found to inhibit growth of cancerous metastatic cells, thereby treating cancer metatstasis.

## Description

The present invention relates to a method for inhibiting and treating cancer metastasis in patients.

Cancer metastasis is the spread of cancer cells that originate from the primary cancer. The diagnosis of metastatic cancers continues to be associated with a death sentence. Often patients die from metastasized cancers originating from the original cancer (also referred to as primary cancer), even though the original (primary) cancer has been successfully treated and cured. As a result, cancer metastasis remains the primary cause of cancer-related deaths. Major obstacles in treatment of cancer metastasis lie in the lack of clinical trials targeting metastasis and the lack of knowledge on the biological processes underlying metastasis. Direct prevention of metastasis and control of metastatic tumor growth is therefore an important step to reduce mortality and improve quality of life of cancer patients.

Metastatic cancer cells have different biological behavior and are genetically and phenotypically often different from the primary cancer cells, which makes treatments for primary cancer cells not necessarily effective for treating metastatic cancer cells. The conventional chemotherapeutics for instance based on Cisplatin, Taxanes, and 5-FU, decease cancer cell growth and proliferation through inhibition of several intracellular mechanisms. However, these drugs do not inhibit cell signaling pathways to prevent the spread of metastatic cancer cells. In metastatic disease these drugs typically inhibit cancer cell growth for several months to one or exceptionally two years, upon which patients present with therapy resistant progressive metastatic disease. Anti-cancer drugs are therefore not necessarily effective against cancer metastasis in cancer patients. The development of primary cancer cells into metastatic cancer cells is controlled by a complex system of intracellular and extracellular signaling and the critical interaction between micro-environmental factors and metastasized cancer cells. As a result, it is challenging to find a specific treatment which would result in preventing and treating cancer metastasis in patients. In order to inhibit or even prevent cancer metastasis, there is a need for compounds that inhibit or even prevent cancer metastasis and that control metastatic cancer growth.

The present invention provides such a compound. It was surprisingly found that anti-BMP4 antibodies could significantly reduce and even prevent migration and invasion of metastasized cancer cells, thereby inhibiting and preventing cancer metastasis of human cancer cells in an animal model. Moreover, in this metastatic murine model the anti-BMP4 antibodies were found to surprisingly inhibit growth of metastasized cancer cells, i.e. inhibit metastatic cancer growth. This proof of principle shows that the antiBMP4 antibodies are extremely suitable for preventing, inhibiting, and/or treating cancer metastasis. Thus, the present invention provides an anti-BMP4 antibody for use in a method of inhibiting, preventing, and/or treating cancer metastasis in patients. The additional advantage of inhibiting growth of metastasized cancer cells further makes the anti-BMP4 antibodies also extremely useful for controlling metastatic cancer growth in cancer patients.

BMP4 is a bone morphogenetic protein (BMP) growth factor that belongs to the TGFβ superfamily. BMPs play important roles in the pathology of several diseases, such as obesity, diabetes, and cancer^{[1]}. The role of BMP4 in cancer metastasis is not exactly known. Several studies indicated that BMP4 has beneficial effects on cancer cells by driving differentiation of these cells and giving rise to less aggressive phenotypes, or by keeping cancer stem cells in a dormant state^{[2]}. Because of the potential anti-tumorigenic effects of BMP4, selective inhibition of BMP4 to prevent metastasis has so far not been deemed as a feasible option. It was therefore even more surprising that inhibition of BMP4 resulted in inhibition and even prevention of cancer metastasis, and inhibition of metastatic cancer growth.

Antibodies against BMP4 have been described. WO2008/030611 describes anti-BMP4 antibodies BMP4. Other antibodies known in the art which bind to BMP4 are for example anti-BMP4 Clone 66119, Mouse IgG2B, R&D, MAB757^{[3]}, anti-BMP4 3H2^{[4]}, and anti-BMP4 EPR6211^{[5]}. Anti-BMP4 antibodies C4, C8 and E7 are described in WO2016/042050 for having an affinity for BMP4. However, none of these antibodies have been suggested for use in the inhibition or prevention of cancer metastasis, nor have they been suggested for use in inhibition of growth of metastatic cancers in patients.

The present invention allows for specific inhibition, prevention, and treatment of cancer metastasis and inhibition of growth of metastatic cancers in patients. The antibodies of the invention are therefore suitable for use in combination with general anti-cancer drugs to ensure that not only the original primary tumor is treated but also metastatic cancer cells.

The anti-BMP4 antibodies according to the invention are suitable for inhibition and prevention, and treatment of cancer metastasis, and inhibition of growth of metastatic disease in patients suffering from cancer. Preferably said cancer is selected from the group consisting of testicular cancer, bone cancer, prostate cancer, mammary cancer, lung cancer, colorectal cancer, breast cancer, gastric cancer, pancreatic, esophageal cancer, ovarian cancer, head and neck cancer, liver cancer, and brain cancer. More preferably, said cancer is esophageal cancer. Most preferred, said esophageal cancer is esophageal adenocarcinoma.

In a preferred embodiment, the anti-BMP4 antibodies according to the invention are particularly suitable for inhibition and/or prevention of cancer metastasis, and inhibition of growth of metastatic tumors wherein the cancer is a SMAD4-deficient cancer. SMAD4 (also known as Mothers Against Decapentaplegic homologue 4 (MADH4) and DPC4) represents the most unique member of the Smad family. SMAD4 is a key regulator within the BMP/SMAD signaling pathway. SMAD4 loss is associated with early disease recurrence and poorer survival. Moreover, SMAD4 deficient tumors often respond less well to chemotherapy. The term "SMAD4 deficient tumor" refers to a tumor characterized by the fact that at least part of its cells have a decreased expression of the SMAD4 gene or have a reduced function of the SMAD4 protein. Deficient tumors may be characterized by comprising at least 10% or more, preferably at least 20% or more, or at least 30% or more of its cells having decreased SMAD4 gene expression or a reduced function of the SMAD4 protein, compared to control cells, or the loss of the SMAD4 protein, by the presence of SMAD4 genomic aberrations (mutations, deletions, etc) which result in a decreased or no expression of the SMAD4 gene or reduced function of the SMAD4protein, or hypermethylation of the SMAD4 locus, or by decreased RNA levels of SMAD4 for instance through siRNAs. Additionally, SMAD4 loss may enhance the metastatic process. BMP4 is an upstream ligand, which via binding to its receptors activates SMAD4. It was therefore all the more surprising that the anti-BMP4 antibodies could be used to inhibit and reduce, and even prevent cancer metastasis from SMAD4-deficient cancers. Typical cancers wherein SMAD4-deficiency results in poor response to therapy and low survival rates are brain cancer, pancreatic cancer, colorectal cancer, gastric cancer, and esophageal cancer ^{[611]}.

Suitable antibodies for use according to the invention is any antibody that has binding affinity to BMP4 and is capable of inhibiting BMP4 signaling. The term "antibody" as used herein refers to any polypeptide comprising an antigen-binding site with at least one complementarity determining region (CDR). The term includes, but is not limited to (i) polyclonal antibodies; (ii) monoclonal antibodies; (iii) monospecific antibodies; (iv) bispecific or multispecific antibodies; (v) humanized antibodies i.e. antibodies in which CDR sequences derived from the germline of another mammalian species have been grafted onto human framework sequences; (vi) chimeric antibodies, i.e. antibodies in which the variable region sequences are derived from one species and the constant region sequences are derived from another species; (vii) human antibodies i.e. antibodies in which both the framework and CDR regions are derived from human germline immunoglobulin sequences; and (viii) single-chain antibodies (ScFv). The term "antibody" also includes antibody fragments or other constructs comprising CDRs that retain antigen-binding function.

The antibodies may be any of the known antibody isotypes and their conformations, for example, IgA, such as IgA1 or IgA2, IgD, IgE, IgG, such as IgG1, IgG2a, IgG2b, IgG3, IgG4, or IgM class, or may constitute mixtures thereof in any combination, such as a mixture of antibodies from the IgG1 and IgG2a class.

The term antibody fragment, as used herein, refers to one or more fragments of an antibody that retain the ability to specifically bind to BMP4 protein (e.g., to an epitope within the BMP4 protein). The antibody fragment may be obtained by manipulation of a naturally-occurring antibody or may be obtained using recombinant methods. Examples of binding fragments encompassed within the term "antigen-binding portion" of an antibody include (i) a Fab fragment i.e. a monovalent fragment consisting of the VL, VH, CL and CH1 domains; (ii) a F(ab')2 fragment i.e. a bivalent fragment comprising two Fab fragments linked by a disulfide bridge at the hinge region; (iii) a Fab' fragment i.e. essentially a Fab with part of the hinge region; (iv) a Fd fragment consisting of the VH and CH1 domains; (v) a Fv fragment consisting of the VL and VH domains of a single arm of an antibody, (vi) a single-domain antibody (sdAb) or nanobody i.e. a fragment consisting of a single monomeric variable antibody domain of the heavy or light chain; and (vii) an isolated complementarity determining region (CDR).

Typical sdAb are derived from the heavy chain antibodies of camelid origin, and consist of one variable domain, termed VHH. Other suitable sdAb can be derived from fish heavy chain antibodies, and consist of a single variable domain, termed VNAR. Other sdAb may be engineered based on common heavy or light chain sequences of an IgG antibody and consist of one variable domain of either the heavy or the light chain. The term sdAb includes those directly isolated from VL, VH, VHH or VNAR reservoir of any origin through phage display or other display technologies and those generated through further modification of such single-domain antibody by humanization, affinity maturation, stabilization, solubilization (e.g., camelization), or other methods of antibody engineering. Also encompassed by the present invention are homologues, derivatives, or fragments that retain or improve the antigen-binding function and specificity of the single-domain antibody.

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv)). Such single chain antibodies are also intended to be encompassed within the term antibody. These antibody fragments are obtained using conventional techniques known to those with skill in the art, and the fragments are screened for utility in the same manner as are intact antibodies.

An anti-BMP4 antibody according to the invention preferably is an IgG, IgE, IgM, Fab, Fab2, scFv, a sdAb or a dimer of a sdAb. More preferably, the anti-BMP4 antibody according to the invention is a sdAb or a dimer thereof. Most preferred anti-BMP4 antibodies according to the invention are VHH antibodies, or VHH dimer antibodies.

The anti-BMP4 antibody according to the invention comprises one or more heavy chains wherein the variable region comprises three complementary determining regions CDR1, CDR2 and CDR3, wherein CDR1 has an amino acid sequence selected from the group of SEQ ID NO: 1, SEQ ID NO: 5, and SEQ ID NO: 9, CDR2 has an amino acid sequence selected from the group of SEQ ID NO: 2, SEQ ID NO: 6, and SEQ ID NO: 10, and CDR3 has an amino acid sequence selected from the group of SEQ ID NO: 3, SEQ ID NO: 7, and SEQ ID NO: 11.

In one embodiment, the anti-BMP4 antibody comprises one or more heavy chains wherein the variable region comprises a CDR1, CDR2 and CDR3 selected from the group of a) a CDR1 consisting of the amino acid sequence of SEQ ID NO: 1, a CDR2 consisting of the amino acid sequence of SEQ ID NO: 2, and a CDR3 consisting of the amino acid sequence of SEQ ID NO: 3, b) a CDR1 consisting of the amino acid sequence of SEQ ID NO: 5, a CDR2 consisting of the amino acid sequence of SEQ ID NO: 6, and a CDR3 consisting of the amino acid sequence of SEQ ID NO: 7, and c) a CDR1 consisting of the amino acid sequence of SEQ ID NO: 9, a CDR2 consisting of the amino acid sequence of SEQ ID NO: 10, and a CDR3 consisting of the amino acid sequence of SEQ ID NO: 11.

Preferably, the anti-BMP4 antibody comprises one or more heavy chains wherein the variable domain comprises a CDR1, CDR2 and CDR3 selected from the group of a) a CDR1 consisting of the amino acid sequence of SEQ ID NO: 1, a CDR2 consisting of the amino acid sequence of SEQ ID NO: 2, and a CDR3 consisting of the amino acid sequence of SEQ ID NO: 3, and b) a CDR1 consisting of the amino acid sequence of SEQ ID NO: 5, a CDR2 consisting of the amino acid sequence of SEQ ID NO: 6, and a CDR3 consisting of the amino acid sequence of SEQ ID NO: 7. More preferably, the anti-BMP4 antibody has one or more heavy chains with a variable domain comprises a CDR1 consisting of the amino acid sequence of SEQ ID NO: 5, a CDR2 consisting of the amino acid sequence of SEQ ID NO: 6, and a CDR3 consisting of the amino acid sequence of SEQ ID NO: 7. Of particular interest are VHH antibodies or dimers thereof wherein the one or more heavy chain variable domain comprises a combination of the aforementioned CDR1, CDR2 and CDR3, more preferably any of the aforementioned CDR combinations.

In another embodiment, the anti-BMP4 antibody comprises one or more heavy chains wherein the variable domain of has an amino acid sequence selected from the group of SEQ ID NO: 4 (C4), SEQ ID NO: 8 (C8), and SEQ ID NO: 12 (E7). Preferably, the anti-BMP4 antibody comprises one or more heavy chains wherein the variable domain has an amino acid sequence selected from the group of SEQ ID NO: 4 or SEQ ID NO: 8, more preferably SEQ ID NO: 8. Particularly suitable are anti-BMP4 VHH antibodies comprising an amino acid sequence of SEQ ID NO: 4 (denoted as C4), SEQ ID NO: 8 (denoted to as C8), or SEQ ID NO: 12 (denoted as E7), preferably SEQ ID NO; 4 or SEQ ID NO: 8, more preferably SEQ ID NO: 8.

Of particular interest are anti-BMP4 antibodies of the invention that comprise two heavy chains with the CDR amino acid sequences or VH amino acid sequences of the invention. The CDR sequences or VH sequences can be identical (homodimers), or different (heterodimers). In one embodiment, the anti-BMP4 antibody is a homodimer, i.e. two heavy chains with identical CDR sequences or identical variable domains sequences according to the invention. Preferably, the homodimer is a VHH homodimer. More preferably, the VHH homodimer is a C4C4 dimer, a C8C8 dimer, or an E7E7 dimer. Even more preferably, the VHH homodimer is a C4C4 dimer or aC8C8 dimer. Most preferably, the VHH homodimer is a C8C8 dimer. In another embodiment, the anti-BMP4 antibody is a heterodimer, i.e. two heavy chains that differ in variable domain. Preferably, the two heavy chains have different variable domains, each variable domain comprising a CDR combination or complete VH amino acid sequence according to the invention. Preferably the heterodimer is a VHH heterodimer, more preferably, a VHH heterodimer selected from a C4C8 dimer, a C8E7 dimer, or a C4E7 dimer. Most preferably, the VHH heterodimer is C4C8.

**Table 1: Amino acid sequences of the CDRs and variable regions**

| | | |
|---|---|---|
| SEQ ID 1 | CDR1 | GRTFSTYA |
| SEQ ID 2 | CDR2 | SKGGGITY |
| SEQ ID 3 | CDR3 | DPVSSVAKSPVAYP |
| SEQ ID 4 | C4 | EVQLVESGGGLVQAGGSLRLSCAASGRTFSTYAMGWTRQAPGKEREFVAAISK GGGITYYSDSVRGRFTISKENAKNTVYLQMNSLKPEDTAVYYCAADPVSSVAKS PVAYPYWGQGTQVTVSS |
| SEQ ID 5 | CDR1 | GRTFRIND |
| SEQ ID 6 | CDR2 | TSGGNTN |
| SEQ ID 7 | CDR3 | DGLRFDSTRYRPFD |
| SEQ ID 8 | C8 | EVQLVESGGGLVQAGGSLRLSCAASGRTFRINDMGWYRQAPGKSRDMVARIT SGGNTNYADSVRGRFTISRDNAKNTVYLQMNSLKPEDTAVYYCNADGLRFDST RYRPFDSWGQGTQVTVSS |
| SEQ ID 9 | CDR1 | GSIRGFVA |
| SEQ ID 10 | CDR2 | TNGGTL |
| SEQ ID 11 | CDR3 | RQIGASGYD |
| SEQ ID 12 | E7 | EVQLVESGGGLVQAGGSLRLSCAASGSIRGFVAMAWYRQAPGKQREWVATVT NGGTLYGDSVRGRFTGSRDNAKNTVYLQMNSLKPEDTAVYYCALRQIGASGYD YWGQGTQVTVSS |

The anti-BMP4 antibodies of the invention can be used in therapy to inhibit and prevent cancer metastasis, and for inhibition of growth of metastasized cancer cells. The anti-BMP4 antibodies of the invention may also be used in combination with other therapeutic drugs, in particular anti-cancer drugs such as cisplatin, Taxanes, 5-FU and the like.

The present invention also provides a method of inhibiting and treating cancer metastasis in cancer patients, comprising administration of an anti-BMP4 antibody according to the invention to a patient suffering from cancer. The antibodies according to the invention can be administered according to any route of administration that is suitable for administering antibodies. Preferably the antibodies are administered via injections. Suitable dosages will be dependent on the route of administration and are generally in the range of 3 to 25 mg/kg body weight, preferably 5 to 20 mg/kg body weight.

The anti-BMP4 antibodies may be produced using any method well known in the art. The antibodies can be produced via standard recombinant techniques, for instance by genetically fusing the CDR sequences or VH sequences into human antibodies to obtain humanized antibodies. VHH antibodies or dimers thereof can be produced using standard recombinant techniques, for example by incorporating the nucleic acid encoding the VHH antibody or dimer thereof into an expression vector, expressing the expression vector in a suitable host cell, and isolating the VHH antibody or dimer thereof.

The anti-BMP4 antibodies according to the invention can be used in pharmaceutical compositions comprising the antibodies according to the invention and a pharmaceutically acceptable carrier. Pharmaceutical compositions comprising antibodies are well known in the art. Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Examples of pharmaceutically acceptable carries are liposomes and (Poly lactic-co-glycolic acid) PLGA (nano) particles. Except insofar as any conventional media or agent is incompatible with the active compound, use thereof in the pharmaceutical compositions of the invention is contemplated.

Therapeutic pharmaceutical compositions typically must be sterile and stable under the conditions of manufacture and storage. The amount of active ingredient which can be combined with a carrier material to produce a single dosage form will vary depending upon the subject being treated and the particular mode of administration. The amount of active ingredient which can be combined with a carrier material to produce a single dosage form will generally be that amount of the composition which produces a therapeutic effect. Generally, out of one hundred per cent, this amount will range from 0.01% to 99% of active ingredient, preferably in the range of from 0.1% to 70%, more preferably in the range from 1% to about 30% of active ingredient in combination with a pharmaceutically acceptable carrier.

In preferred embodiment, the pharmaceutical composition may further comprise an anti-cancer drug. Anti-cancer drugs, such as platinum, Taxanes and/or 5-FU complexes or derivates are known to be useful for the treatment of different kinds of cancers. Exemplary kinds of cancer include, but are not limited to lung cancer, testicular cancer, breast cancer, colon cancer, ovarian cancer, head and neck cancer, esophageal cancer, or gastric cancer. All of these cancer types are known to be treatable with anti-cancer drugs based on or including platinum, Taxanes and/or 5-FU complexes or derivates and are also known to develop therapy resistance against the anti-cancer drugs used to treat these types of cancers. In a preferred embodiment, said anti-cancer drug comprises platinum complexes, more preferably cisplatin. In a preferred embodiment, the pharmaceutical composition comprises an anti BMP4 antibody according to the invention in combination with cisplatin.

### EXAMPLES

### Legends of Figures

Figure 1: The timelines indicate the treatment periods with C4C4 (VHHs) with or without cisplatin during follow up of the mice (days) until humane endpoints. The maximum time of follow up was 84 days.
Figure 2: Ex vivo imaging of luciferase activity indicating the luciferase activity of the metastasized Luciferase-eGFP-SMAD4(-) ISO76P cells in the lungs of each mouse at the day of culling. Arrows indicate lesions in the lungs with flux levels > 0.5 × 10⁶.
Figure 3: Quantification of the relative luciferase activity of the metastasized Luciferase-eGFP-SMAD4(-) ISO76P cells in the lungs of each mouse at the day of culling of mice treated with C4C4 for 4 weeks, with or without 4 weeks of treatment with cisplatin. (Dashed lines refer to 1×10⁶ flux level).
Figure 4: The timelines indicate the treatment periods with C8C8 and cisplatin during follow up of the mice (days) until humane endpoints.
Figure 5: (A) Quantification of the relative luciferase activity of the metastasized Luciferase-eGFP-SMAD4(-) ISO76P cells in the lungs of each mouse at the day of culling of mice treated with C8C8 with or without 4 weeks of treatment with cisplatin. (Dashed line refer to 1×10⁶ flux level). (B) Analysis of the gene expression of the ratios of human versus mouse Vimentin expression in the lungs of each mouse. A ratio > 0.2 was considered as positive for the presence of human metastatic cells.
Figure 6: (A) Quantification of the relative luciferase activity of the metastasized Luciferase-eGFP-SMAD4(-) ISO76P cells in the liver of each mouse at the day of culling. (B) Analysis of the gene expression of the ratios of human versus mouse Vimentin expression in the liver of each mouse. A ratio > 0.2 was considered as positive for the presence of human metastatic cells.
Figure 7: A dose response curve and analysis indicating the association between an increasing dose of C8C8 compared to the presence and burden of lung metastasis in mice.
Figure 8: A dose response curve and analysis indicating the association between an increasing dose of C8C8 compared to the presence and burden of liver metastasis in mice.

### Example 1

A patient derived metastatic tumor xenograft (PDX) model was established using the esophageal adenocarcinoma (EAC) human cancer cell line ISO76P in NOD-SCID IL-2RγKO (NSG) mice. The ISO76P cell line was derived from the primary cancer of a patient with EAC^{[12]}. The ISO76P cell line was found to carry a SMAD4 mutation^{[13]}. In this in vivo study the mice were inoculated subcutaneously with the ISO76P cells. Cells were prepared in 50%: 50% Matrigel: PBS mix and injected subcutaneously into the right flank of the NSG mice, (2.5 × 10⁶ cells/mice in 100 uL suspension). Once tumors reached a volume of > 80 mm³ treatments were initiated.

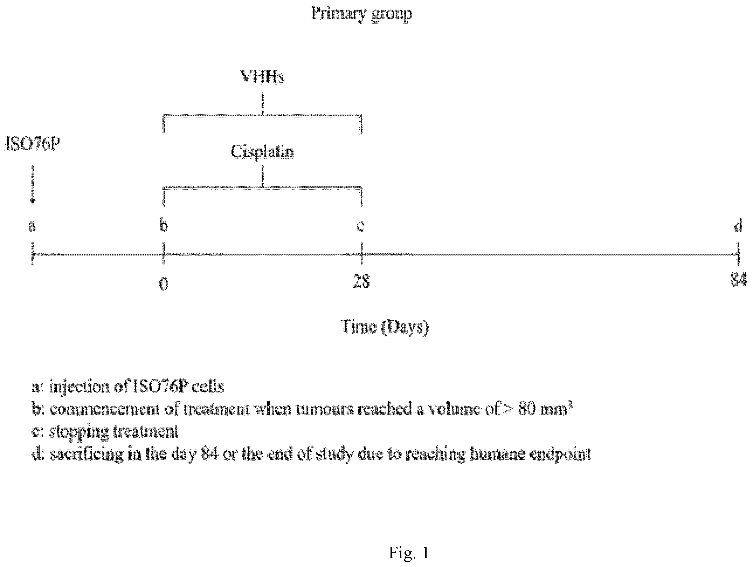

In this experiment the potential of C4C4 (anti-BMP4) on the occurrence of metastasis was tested as proof of principle. Hereto, mice were treated with the C4C4 with or without Cisplatin. A total of 30 mice aged 6 to 8 weeks were inoculated with the ISO76P cells and randomized into four subgroups of 5 mice each. Subgroup 1 received vehicle (0.9% NaCl saline); Subgroup 2 received Cisplatin 2 mg/kg once per week; Subgroup 3 received daily 100 µg C4C4 via intraperitoneal injections (IP) and Subgroup 4 received Cisplatin 2 mg/kg once per week in combination with C4C4, 100 µg daily IP. All formulations were dissolved in saline. The duration of treatments was 28 days (Figure 1). Animals were left for a maximum of 12 weeks or reaching a humane endpoint (tumor > 1.5 cm³, ascites, weight loss > 20%). After sacrificing the mice, luciferase imaging for the mice and for the organs including, large and small bowel, stomach, esophagus, lung, liver, spleen, brain, and any site bearing tumor metastasis was performed to detect metastases. Imaging of the tumor and metastatic lesions in PDXs was possible as the tumor cells carried a Luciferase-eGFP+ construct.

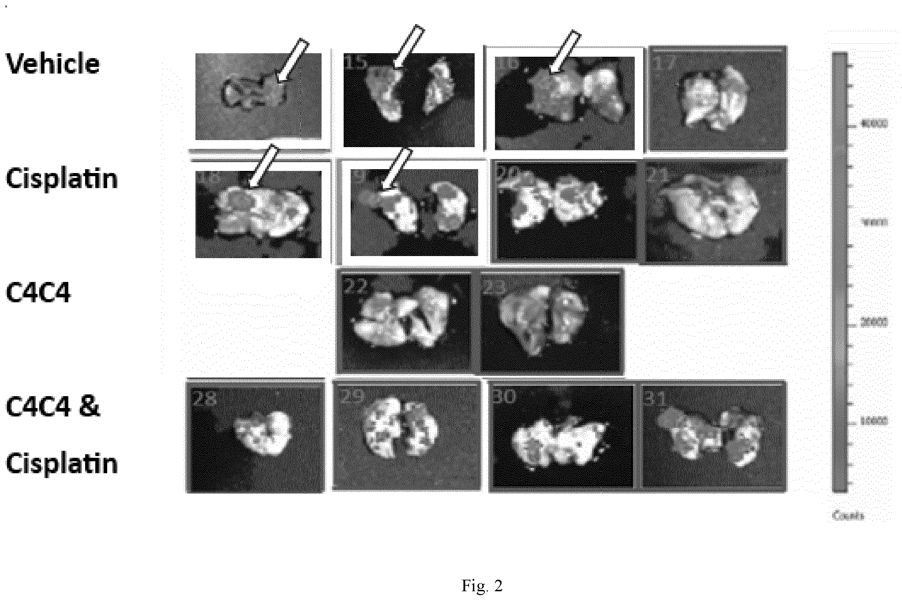

Imaging was performed by using the Xenogen IVIS 100 Imaging System (Caliper, Life Science). The imaging was used for detection and quantification of the tumor xenografts and organ metastases. 100 µL of 20 mg/ml luciferin (Promega) in PBS was subcutaneously injected into each mouse 5 min before imaging. At the study endpoint, the whole mouse and its organs were imaged to determine the tumor burden (Figure 2). The imaging exposure times were 60 s for whole animal and 5 min for organs. The bioluminescence signal was quantified using in Living Image software.

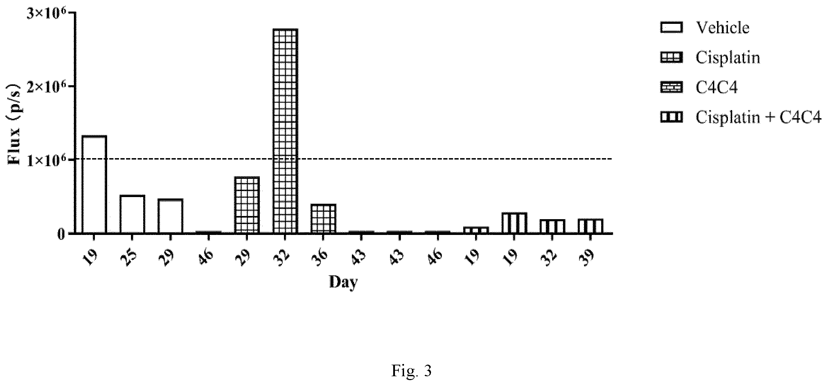

Eight mice were lost due to not outgrowing the PDX and could not be included in the analysis. Mice reached human endpoints after an average follow up of 35 days (range 30-45 days). Both groups treated by C4C4 alone and C4C4 in combination with cisplatin did not show the formation of organ metastasis, while lung metastasis were observed in the vehicle and cisplatin groups (Figure 2). In these groups five out of eight animals had increased flux values, (flux values > 0.5 ×10⁶), indicating metastasis (Figure 3). Animals treated with C4C4 had significantly less frequent development of metastasis compared to the two other groups (p = 0.05; Chi-square test). In sum, treatment of a metastatic PDX model with the ISO76P EAC cell line with C4C4, in a dose of 100 ug 5 days per week for 4 weeks, with or without cisplatin treatment in a dose of 2 mg/kg/week for 4 weeks, prevented the formation of metastasis during an average follow up period of 35 days when compared to treatment with cisplatin alone or vehicle.

### Example 2

A patient derived metastatic tumor xenograft (PDX) model was established by using the esophageal adenocarcinoma (EAC) human cancer cell line ISO76A in NOD-SCID IL-2RγKO (NSG) mice to test the effect of C8C8 on the formation of metastasis. This highly metastatic ISO76A cell line was derived from metastasized cells of a patient with EAC ^{[12]}. The ISO76A cell line was found to carry a SMAD4 mutation ^{[13]}. In this in vivo study the mice were inoculated subcutaneously with the ISO76A cells. The ISO76A cells were prepared in 50%: 50% Matrigel: PBS mix and injected subcutaneously into the right flank of the NSG mice, (2.5×10⁶ cells/mice in 100 uL suspension). Once tumors reached a volume of > 80 mm³ treatments were initiated.

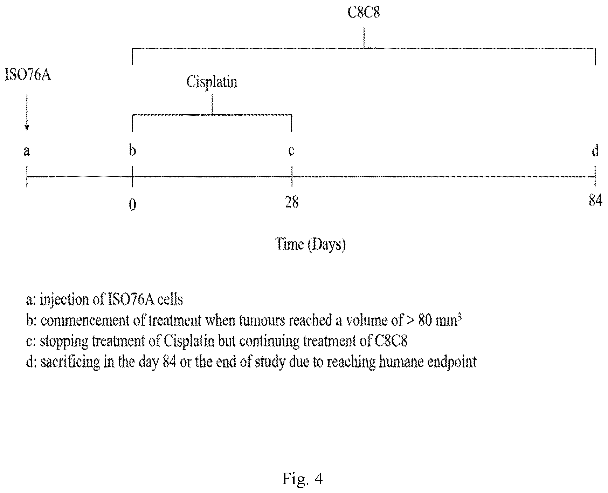

As proof of principle, 25 mice were randomized into five groups of five mice each, which were treated with an increasing total dose of C8C8. Group 1 received 2 mg/kg of Cisplatin once per week for 4 weeks; Group 2 received 2 mg/kg of Cisplatin once per week for 4 weeks + 8 mg/kg of C8C8 as a single dose once per week until sacrifice; Group 3 received 2 mg/kg of Cisplatin once per week for 4 weeks + 8 mg/kg of C8C8 as a single dose per day (3 days/week, Mon/Wed/Fri) until sacrifice; Group 4 received 2 mg/kg of Cisplatin once per week for 4 weeks + 8 mg/kg/day of C8C8 as a single dose per day (5 days/week) until sacrifice; Group 5 received 2 mg/kg of Cisplatin once per week for 4 weeks + 8 mg/kg of C8C8 as 2 doses of 4 mg/kg per day (5 days/week) until sacrifice. In case of an animal could not participate in the study due to no outgrowth of the PDX, the animal was replaced. Animals were sacrificed when they received treatments for a maximum of 12 weeks or when reaching a humane endpoint (Figure 4). Human endpoints were tumor > 1.5 cm³, ascites, weight loss > 20%. After sacrificing the mice, luciferase imaging for organs was performed to detect metastases. Ex vivo imaging of the metastatic lesions in PDXs was possible as the tumor cells carried a Luciferase-eGFP+ construct.

Imaging was performed by using the Xenogen IVIS 100 Imaging System (Caliper, Life Science). The imaging was used for detection and quantification of the tumor xenografts and organ metastases. 100 µL of 20 mg/ml luciferin (Promega) in PBS was subcutaneously injected into each mouse 5 min before imaging. At the study endpoint, the whole mouse and its organs were imaged to determine the tumor burden. The imaging exposure times were 60 s for whole animal and 5 min for organs. The bioluminescence signal was quantified using Living Image software.

The ISO76-A cells are known to expresses Vimentin, therefore the metastatic lesions in the lung and liver were confirmed by the expression of human Vimentin. The gene expressions of human and mouse Vimentin in the metastatic tissues was detected by qPCR with TaqMan probes (Thermofisher) according to the manufacturer's instructions. In the experimental settings, FAM and VIC were used as 5' fluorescent reporter dye. TAMRA was used as 3' quencher dye. The sequences for two sets of probes and primers are from conserved regions of human and mouse genomes coding for Vimentin protein. Amplification was performed in 12 µL reaction volume containing 6 µL of 2x mastermix (Fast universal master mix, Thermofisher), 0.5 µL of forward primers of Vimentin (mouse), 0.5 µL of reverse primers of Vimentin (mouse), 0.5 µL of forward primers of Vimentin (human), 0.5 µL of reverse primers of Vimentin (human), 1 µL of Taqman probe of Vimentin (mouse), 1 µL of Taqman probe of Vimentin (human) and 2 µL of DNA (mouse + human). The reactions were carried out on a Roche Light Cycler 480. A sharp rise of the "s-shape" curve was considered to be positive amplification compared with the relative horizontal line. The triplicate cycle threshold values were used for statistical analysis. Ratios between mouse and human Vimentin were calculated. A ratio > 0.2 was regarded as positive for the presence of metastatic human cancer cells.

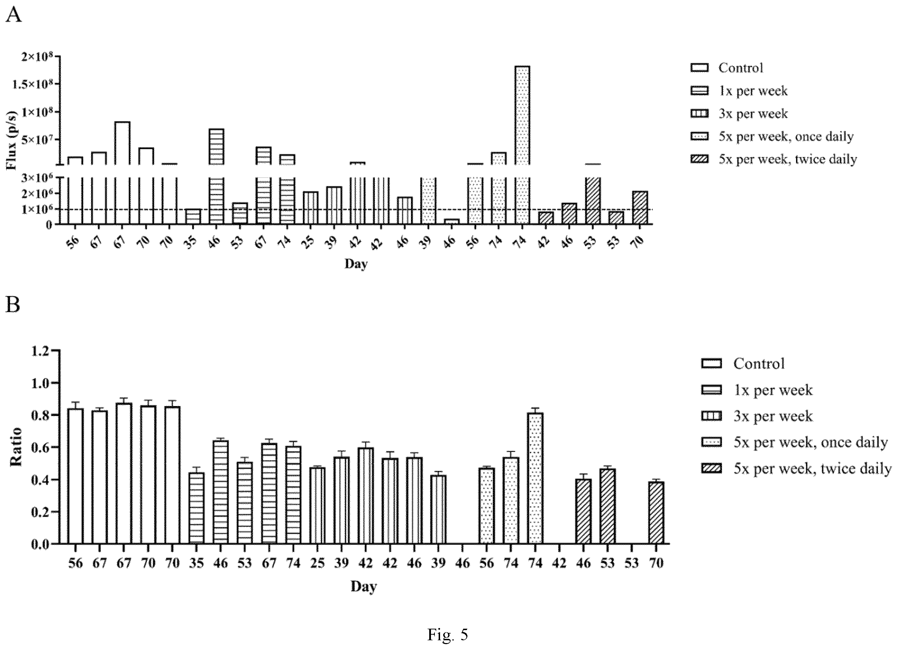

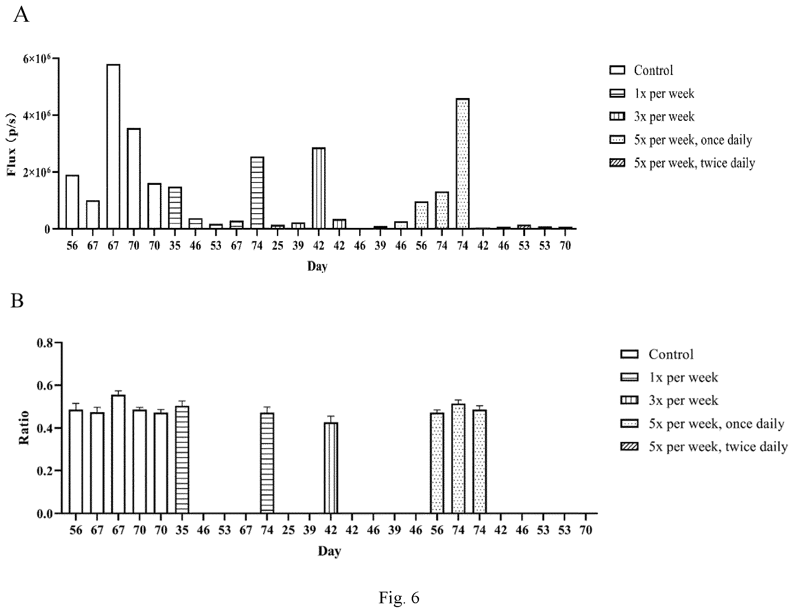

Continued treatment with C8C8 for 5 times per week at a dose of 4 mg/kg/twice a day, showed reduced formation of both, lung and liver metastases by 40% (Figures 5 and 6; p<0.01, Mann-Whitney U test). Interestingly, this treatment showed a reduction of liver metastasis by a 100% (Figure 6; p<0.001, Mann-Whitney U test). Thus, none of the animals treated 5 times per day at a dose of 4 mg/kg/twice per day, developed liver metastasis, while overall there was a 40% reduction of lung and liver metastases (Figures 5 and6).

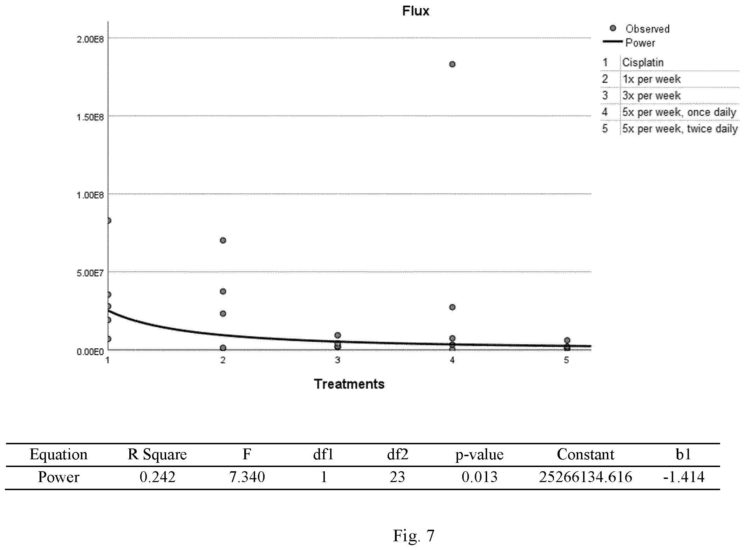

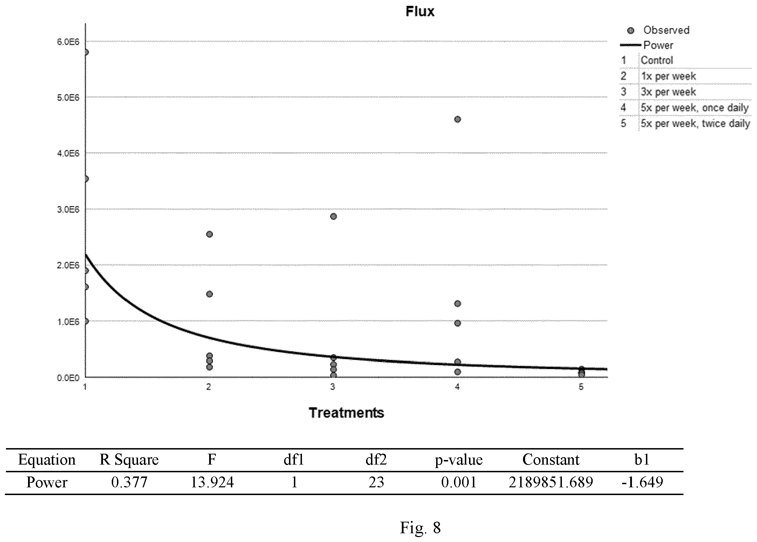

Logistic regression analysis indicated that therapy by C8C8 significantly decreased the burden of both lung and liver metastasis in a dose dependent way (Figure 7 and 8; p=0,01 and 0.001 resp). In sum, therapy by C8C8 to prevent and treat metastasis is dose dependent. Treatment for 5 days per week of 4mg/kg/two times per day significantly decreased the burden and formation of both lung and liver metastasis in the ISO76A PDX model.

### REFERENCES

1. Bragdon et al. Cell Signaling (2011) 23(4) : 609-20 (Doi :10.1016/j.cellsign.2010.10.003
2. Fares et al. Signal Transduction and Targeted Therapy (2020) 5:28. (doi: 10.1038/s41392-202-0134-x
3. Khurana et al. Stem Cells, Vol. 32, no. 11, 14 November 2014 (2014-11-14), pages 3012-3022
4. Prepotech, 500-M121, described in A. Bhattacherjee et al., Journal of Neuroscience, vol. 33, no. 3, 16 January 2013 (2013-01-16), pages 1050-1061
5. Millipore, MABD188, described in Kwak Young-Don et al., Biochemical and Biophysical Research Communications, vol. 447, no. 3, 13 April 2014 (2014-04-13), pages 394-39
6. Shugang et al, Transl oncol, 2016 feb; 9 (1) 1-7
7. Shugang et al, clin cancer res 2009 jul 5:15(14):4674) (PMID 19584151);
8. Yoo et al, J Pathol Transl Med. 2019 Sep; 53(5): 289-297. doi: 10.4132/jptm.2019.06.07
9. Wang et al, Clin Cancer Res . 2007 Jan 1;13(1):102-10. doi: 10.1158/1078-0432.CCR-06-1467
10. Singhi et al, Am J Surg Pathol. 2015 Apr; 39(4): 487-495. doi: 10.1097/PAS.0000000000000356
11. He et al, J Exp Clin Cancer Res. 2011; 30(1): 70. doi: 10.1186/1756-9966-30-70
12. Read et al, Ann Surg Oncol. 2016 Jan;23(1):305-11. doi: 10.1245/s10434-015-4425-3
13. Li et al, Cell Oncol. 2022 Aug;45(4):639-658. doi: 10.1007/s13402-022-00689-2

## Claims

1. Anti-BMP4 antibody for use in a method of preventing, inhibiting, and/or treating cancer metastasis in patients.

2. Anti-BMP4 antibody for use according to claim 1, wherein said cancer is selected from the group consisting of testicular cancer, bone cancer, prostate cancer, mammary cancer, lung cancer, colorectal cancer, breast cancer, gastric cancer, pancreatic, esophageal cancer, ovarian cancer, head, neck cancer, liver cancer, and brain cancer.

3. Anti-BMP4 antibody for use according to claim 2, wherein said cancer is esophageal cancer.

4. Anti-BMP4 antibody for use according to claim 3, wherein said esophageal cancer is esophageal adenocarcinoma.

5. Anti-BMP4 antibody for use according to any one of the proceeding claims, wherein said cancer is a SMAD4-deficient cancer.

6. Anti-BMP4 antibody for use according to any one of the proceeding claims, wherein the anti-BMP4 antibody is selected from the group consisting of IgG, IgE, IgM, Fab, Fab2, scFv and VHH antibodies.

7. Anti-BMP4 antibody for use according to any one of the preceding claims, wherein the anti-BMP4 antibody is a VHH antibody or dimer thereof.

8. Anti-BMP4 antibody for use according to any one of the preceding claims, wherein the variable domain one or more heavy chains (VH) of the antibody comprises a CDR1, CDR2 and CDR3 selected from the group of:
a. a CDR1 consisting of the amino acid sequence of SEQ ID NO: 1, a CDR2 consisting of the amino acid sequence of SEQ ID NO: 2, and a CDR3 consisting of the amino acid sequence of SEQ ID NO: 3,
b. a CDR1 consisting of the amino acid sequence of SEQ ID NO: 5, a CDR2 consisting of the amino acid sequence of SEQ ID NO: 6, and a CDR3 consisting of the amino acid sequence of SEQ ID NO: 7, and
c. a CDR1 consisting of the amino acid sequence of SEQ ID NO: 9, a CDR2 consisting of the amino acid sequence of SEQ ID NO: 10, and a CDR3 consisting of the amino acid sequence of SEQ ID NO: 11.

9. Anti-BMP4 antibody for use according to any one of the preceding claims, wherein the variable domain one or more heavy chains (VH) of the antibody comprises a CDR1, CDR2 and CDR3 selected from the group of:
a. a CDR1 consisting of the amino acid sequence of SEQ ID NO: 1, a CDR2 consisting of the amino acid sequence of SEQ ID NO: 2, and a CDR3 consisting of the amino acid sequence of SEQ ID NO: 3, and
b. a CDR1 consisting of the amino acid sequence of SEQ ID NO: 5, a CDR2 consisting of the amino acid sequence of SEQ ID NO: 6, and a CDR3 consisting of the amino acid sequence of SEQ ID NO: 7.

10. Anti-BMP4 antibody for use according to any one of the preceding claims, wherein the variable domain (VH) of one or more heavy chains of the antibody comprises a CDR1 consisting of the amino acid sequence of SEQ ID NO: 5, a CDR2 consisting of the amino acid sequence of SEQ ID NO: 6, and a CDR3 consisting of the amino acid sequence of SEQ ID NO: 7.

11. Anti-BMP4 antibody for use according to claims 1-8, wherein the variable domain of one or more heavy chains comprises an amino acid sequence selected from the group of SEQ ID NO: 4 (C4), SEQ ID NO: 8 (C8), and SEQ ID NO: 12 (E7).

12. Anti-BMP4 antibody for use according to claim 1-9, wherein the variable domain of the one or more heavy chain of the antibody comprises the amino acid sequence of SEQ ID NO: 4 or SEQ ID NO: 8.

13. Anti-BMP4 antibody for use according to claims 1-10, wherein the heavy chain of the variable domain of the one or more heavy chains comprises the amino acid sequence of SEQ ID NO: 8.

14. Anti-BMP4 antibody for use according to claims 1-13, wherein the anti-BMP4 antibody is a VHH homodimer.

15. Anti-BMP4 antibody for use according to claims 1-13, wherein the anti-BMP4 antibody is a VHH heterodimer.

16. A method of inhibiting and treating cancer metastasis in cancer patients, comprising administration of an anti-BMP4 antibody according to any one of the preceding claims.
